# EUROPEAN PATENT SPECIFICATION

(11) **EP 1 562 531 B1**
(45) Date of publication and mention of the grant of the patent: **13.01.2016**
(21) Application number: 03776536.9
(22) Date of filing: 24.10.2003
(51) Int. Cl.: A61K 9/06, A01N 25/00, A61K 31/05, A61K 8/63, A61K 8/67, A61K 8/69, A61K 31/58, A61Q 19/02

(54) **TOPICAL SKIN CARE COMPOSITION**
TOPISCHE HAUTPFLEGEZUSAMMENSETZUNG
COMPOSITION DE SOIN POUR LA PEAU A APPLICATION TOPIQUE

(30) Priority: 25.10.2002 US 280483
(43) Date of publication of application: 17.08.2005
(60) Divisional application: 15189356.7
(73) Proprietor: Galderma S.A., 6330 Cham (CH)
(72) Inventor: PUGLIA, Nancy, c/o HILL DERMACEUTICALS, INC., Sanford, FL 32773 (US); RAMIREZ, Rosario, c/o HILL DERMACEUTICALS, INC., Sanford, FL 32773 (US); ROTH, Jerry, c/o HILL DERMACEUTICALS, INC., Sanford, FL 32773 (US)
(74) Representative: Brasnett, Adrian Hugh
(86) International application number: PCT/US2003/033876
(87) International publication number: WO 2004/037201

(56) References cited:
- WO-A1-97/03648
- US-A- 3 856 934
- US-A- 4 489 071
- US-A- 5 538 737
- US-A- 5 656 672
- US-A- 5 660 837
- US-A- 5 976 555
- US-A- 6 080 393
- CLARK J: "CREAM COMBO CALLED MELASMA 'DRUG OF CHOICE'", DERMATOLOGY TIMES, ADVANSTAR COMMUNICATIONS, CLEVELAND, OH, US, vol. 23, no. 5, 1 May 2002 (2002-05-01), page 1/02, XP008062911, ISSN: 0196-6197

## Description

### FIELD OF THE INVENTION

The invention relates generally to a method of making a medicated skin treating composition.

### BACKGROUND OF THE INVENTION

Melasma or chloasma is a common pigmentary condition that affects primarily women in their reproductive years. Dark, mottled (hyperpigmented) patches appear on the face and neck, especially on the cheeks and forehead. Melasma is usually triggered by hormonal activity that is the result of pregnancy or birth control pills. Thus, the condition is known as the "mask of pregnancy." The condition occurs when excess melanin is deposited in the cells of the epidermis and dermis. Melasma can persist for long periods of time and often recurs with subsequent pregnancies. The condition is less common among men, who account for about 10% of all cases.

Standard therapy involves depigmenting, or bleaching, the affected areas of the skin, the use of sunscreens, and avoidance of sunlight. Hydroquinone is the most popular topical depigmenting agent. Concentrations of 5%-10% hydroquinone are very effective, but can be irritating. The chemical stability of hydroquinone formulations is important because hydroquinone is easily oxidized and loses potency. The most commonly used agent usually involves a 16-week to 20-week course of therapy, and some therapies can take longer. Tretinoin (Retin-A) is another widely used therapy for melasma.

Nevertheless, there remains a need in the art for a therapeutic approach that would contain several medicines for the treatment of melasma in a single composition. Moreover, it would be useful to have a therapeutic carrier, such as a cream, that would facilitate the penetration of the medicaments into the skin.

U.S. Pat. No. 5,538,737 discloses a method of making a water-in-oil emulsion containing a pharmaceutically acceptable salt of an H₂-antagonist. The steps include dissolving the pharmaceutically acceptable salt in an aqueous medium to form a water portion; combining the water portion with an oil portion, comprising an edible oil comprising an ester or mixed ester of glycerol and an emulsifying agent to form a water portion and oil portion matrix; then emulsifying the matrix to form the water-in-oil emulsion.

U.S. Pat. No. 5,656,672 discloses a process for preparing a water-in-oil emulsion with retinal as the active ingredient. The emulsion contains an oil phase including at least one organic solvent for retinal (such as aliphatic fatty alcohols) and optional lipophilic additives; an aqueous phase containing water and optional hydrophilic additives; and an agent for emulsifying the aqueous phase in the oil phase. The oil phase and the aqueous phase are independently prepared, and the aqueous phase is incorporated into the oil phase, with subsequent addition of a phase-containing retinol and its solvent.

U.S. Pat. No. 5,660,837 discloses a process for the preparation of a pharmaceutical formulation in the form of an oil-in-water emulsion. The steps of the process include of adding the emulsion-stabilizing surface active drug and an optimal conventional surfactant to a two-phase, oil-water system at room temperature; allowing the emulsion-stabilizing surface active drug to equilibrate at an interface; adding an agent giving isotonicity to the final formulation; and homogenizing by high pressure technique.

U.S. Pat. No. 5,976,555 discloses skin care compositions. An oil-in-water emulsion base contains retinoids; cetearyl alcohol and cetearyl glucoside or a mixture of a polyethylene glycol ethers of stearyl alcohol; cetyl alcohol, stearyl alcohol and mixtures thereof; a light, dry absorbable oil; and substantive, emollient oils or waxes.

Clark et al., Dermatology Times 23(5), page 1 (2002) describes a topical cream combining 4% hydroquinone, 0.05% tretinoin and 0.01% fluocinolone acetonide for use in treating melasma.

U.S. Pat. No. 6,080,393 discloses a skin care composition comprising an oil-in-water emulsion with a therapeutically effective amount of a retinoid; wherein the oil phase comprising one or more oils, and an effective amount of at least one oil-soluble antioxidant; and wherein the composition comprises a corticosteroid.

Nevertheless, there remains a need in the art for a method of making a smoother cream base for the application of therapeutic agents for the treatment of melasma, which will facilitate the penetration of the medicaments into the skin.

### SUMMARY OF THE INVENTION

The invention provides a method of making a topical medicated composition, as defined in claim 1.

The process for making the cream base entails (a) mixing the hydrophilic compounds with water to form an aqueous phase; (b) mixing the hydrophobic compounds to form a hydrophobic (non-aqueous or wax) phase; then (c) mixing the hydrophilic and hydrophobic phases with one another to form a biphasic mixture; and finally (d) adding an emulsifier to the biphasic mixture to form the emulsion. By mixing the emulsifier after the aqueous and non-aqueous phases have been mixed, the result is a smoother-textured cream that disappears on application to skin, as compared to creams made by processes where the emulsifier is added to the aqueous or non-aqueous phases earlier in the process. Because the emulsifier is added as the final step, less wax is needed in making the cream, resulting in a "thinner" hydrophilic cream that disappears faster when applied to the skin, as compared to creams made by processes where the emulsifier is added to the aqueous or non-aqueous phases earlier in the process.

The cream base made by the method of interest can be a carrier for any of a variety of pharmaceutically active agents for dermatologic use. For example, anti-acne, anti-cancer, antibiotic, anti-inflammatory, hormone, anti-fungal and analgesic active agents can be incorporated into a cream base of interest.

In this invention, the cream base comprises a steroid, a keratolytic agent and a depigmenting agent, wherein the steroid is fluocinolone acetonide, the keratolytic agent is tretinoin and the depigmenting agent is hydroquinone.

In a more specific embodiment, the cream also includes the inactive ingredients butylated hydroxytoluene, cetyl alcohol, citric acid, glycerin, glyceryl stearate, magnesium aluminum silicate, methyl gluceth-10, methylparaben, PEG-100 stearate, propylparaben, purified water, sodium metabisulfite, stearic acid and stearyl alcohol.

The cream can be Tri-Luma^{®} Cream, which is the first approved product to combine the standard depigmenting agent, hydroquinone, with tretinoin and a topical low-potency steroid that can be applied as a single preparation. The recommended course of therapy for Tri-Luma^{®} Cream is 8 weeks, and significant results have been seen after the first 4 weeks of treatment. Another advantage of the process of the invention is that by controlling the temperature at which the components, including hydroquinone, are added, the cream does not turn as brown, resulting in a more pleasing-colored product.

### DETAILED DESCRIPTION OF THE INVENTION

Creams are emulsions of hydrophilic and lipophilic (hydrophobic) components. Generally, an emulsifier or surface active agent is included to enhance the mixing of the reagents resulting in a stable emulsion.

The various compounds that comprise an inert carrier are generally known in the art. By "inert" is meant not having a pharmacologic activity. Typical examples of inert compounds comprising a cream base include cetyl alcohol, lanolin, glycerin, ethanol, EDTA, methyl paraben, zinc oxide, titanium dioxide, benzoic acid, carboxymethylcellulose, dimethylsulfoxide, polyethyleneglycol, petroleum, citric acid and stearic acid.

The instant invention relates to a method of making a cream base as a vehicle for one or more pharmacologically active agents for dermatologic applications. The method of interest comprises a particular order of adding and mixing of the ingredients of a cream. The hydrophilic ingredients, including water, are mixed. Heating may be used to facilitate dissolving and solubility to produce a solution. The lipophilic or hydrophobic ingredients are mixed separately. Heating may be used to facilitate mixing and homogenization.

The hydrophilic solution and the lipophilic solution then are mixed and blended. One or more pharmaceutically active agents then are added to the blended mixture. Then, one or more emulsifiers are added and the entire mixture blended to produce a dermatologic cream of interest.

The temperatures for heating the hydrophobic and hydrophilic solutions is that sufficient to facilitate the obtention of a homogeneous solution. Generally, a lower elevated temperature with longer mixing time is preferred. The temperature also may be limited by the properties of any one of the individual ingredients therein. Generally, the temperature does not exceed about 100°C. Preferably, the temperature does not exceed about 90°C or about 80°C or about 70°C or about 60°C. Generally, the temperature of heating need not be exact, at least within the accuracy of standard temperature measurement means.

The hydrophobic and hydrophilic solutions need not be heated to the same temperature. Having the same temperature facilitates the mixing of the two solutions. If the solutions are at different temperatures, the warmer solution is cooled to the temperature of the cooler solution prior to mixing.

The blended mixture optionally may be cooled prior to mixing in the one or more pharmacologically active agent or agents. The physical properties of the active agents may dictate a need for cooling.

Following that step and blending, one or more emulsifiers are added. The mixture is blended thoroughly to produce a cream of interest. If elevated, the temperature can be reduced during the blending.

As to the lipophilic ingredients, as known in the art, oils may be derived from animals, plants, nuts, petroleum etc. Those derived from animals, plant seeds and nuts are similar to fats and consequently, may contain a significant number of polar acid and/or ester groups. Alternatively, oils derived from petroleum are usually aliphatic or aromatic hydrocarbons that are essentially free of polar substitution.

Oil-based products which can be used include hydrocarbons or mineral fats obtained by the distillation of petroleum (petroleum jelly); vegetable oils and liquid triglycerides; animal fats or solid, natural triglycerides; and waxes or solid ethers of fatty acids, such as stearic acid and palmitic acid, and organic alcohols. Lanolin or wool fats made of fatty acids and cholesterol esters; and cetyl and stearyl alcohols, which are solid alcohols obtained by hydrogenation of their respective acids are also useable. Amphoteric compounds such as soaps or salts of fatty acids that may be acidic or basic depending on whether the lipophilic group is anionic or cationic, sulfated alcohols which are semi-synthetic substances and synthetic surface active agents are known in the art and also can be used. Glycerin is obtained from fats and, due to the hydrophobicity thereof, has the property of extracting water from the surface of mucosa or denuded skin. Glycerin does not damage intact skin because of having hydrophilic properties, and is a useful humectant.

Other materials that may be used in a topical preparation of interest include liquid alcohols, liquid glycols, liquid polyalkylene glycols, liquid esters, liquid amides, liquid protein hydrosylates, liquid alkylated protein hydrosylates, liquid lanolin and lanolin derivatives and other like materials. Particular examples include monohydric and polyhydric alcohols, e.g ., ethanol, isopropanol, glycerol, sorbitol, 2-methoxyethanol, diethylene glycol, ethylene glycol, hexylene glycol, mannitol, cetyl alcohol and propylene glycol; ethers such as diethyl or dipropyl ether; polyethylene glycols and methoxypolyoxyethylenes; carbowaxes having molecular weights ranging from 200 to 20,000; polyoxyethylene glycerols; polyoxyethylene; sorbitols; and stearoyl diacetin.

The topical carriers often include both an alcohol and water so as to accommodate lipophilic and hydrophilic components. Other ingredients include buffers, such as sodium hydroxide, sodium citrate or tetrasodium EDTA; excipients; fragrances such as menthol; opacifiers such as zinc oxide, magnesium aluminum silicate and titanium dioxide; preservatives such as dichlorobenzyl alcohol, benzoic acid, methylparaben and phenyl carbinol; antioxidants; gelling agents such as petrolatum and mineral wax; thickening agents such as carboxymethylcellulose; stabilizers; surfactants; emollients; coloring agents and the like.

In addition, the topical carrier may include a penetration enhancer defined as a material that increases the permeability of the skin to one or more active agents so as to allow for cutaneous delivery of a pharmacologically active agent. Various compounds for enhancing the permeability of skin are known in the art. For example, dimethylsulfoxide (DMSO), dimethyl formamide (DMF) and N,N-dimethylacetamide (DMA), decylmethylsulfoxide, polyethylene glycol monolaurate and the 1-substituted azacycloheptan-2-ones.

A number of different emulsifiers or surfactants can be used to prepare a topical preparation of interest. Nonlimiting examples of amphoteric surfactants useful in the compositions of the present invention are disclosed in McCutcheon's, "Detergents and Emulsifiers", North American edition (1986) and McCutcheon's, "Functional Materials", North American edition (1992). Surfactants that can used are the betaines, sultaines and hydroxysultaines. Examples of betaines include the higher alkyl betaines, such as coco dimethyl carboxymethyl betaine, lauryl dimethyl carboxymethyl betaine, lauryl dimethyl alphacarboxyethyl betaine, cetyl dimethyl carboxymethyl betaine, cetyl dimethyl betaine, lauryl bis-(2-hydroxyethyl) carboxymethyl betaine, steryl bis-(2-hydroxypropyl) carboxymethyl betaine, oleyl dimethyl gamma-carboxypropyl betaine, lauryl bis-(2-hydroxypropyl)alpha carboxyethyl betaine, coco dimethyl sulfopropyl betaine, stearyl dimethyl sulfopropyl betaine, stearyl betaine, lauryl dimethyl sulfoethyl betaine, lauryl bis-(2-hydroxyethyl) sulfopropyl betaine, amidobetaines, amidosulfobetaines, oleyl betaine and cocamidopropyl betaine. Examples of sultaines and hydroxysultaines include cocamidopropyl hydroxysultaine. Examples of other amphoteric surfactants are alkyliminoacetates, iminodialkanoates and aminoalkanoates.

Examples of anionic surfactants also are disclosed in McCutcheon's, "Detergents and Emulsifiers", North American edition (1986) and McCutcheon's, "Functional Materials", North American edition (1992). Examples include the alkoyl isothionates, the alkyl and alkyl ether sulfates, such as, ammonium cocoyl isothionate, sodium cocoyl isothionate, sodium lauroyl isothionate, sodium stearoyl isothionate and mixtures thereof, the sarcosinates, such as sodium lauroyl sarcosinate, sodium cocoyl sarcosinate and ammonium lauroyl sarcosinate, sodium lauryl sulfate, ammonium lauryl sulfate, ammonium cetyl sulfate, sodium cetyl sulfate, sodium stearyl sulfate, ammonium cocoyl isethionate, sodium lauroyl isethionate, sodium lauroyl sarcosinate and mixtures thereof.

Other emulsifiers includes tricetareth-4-phosphate, sodium laureth-4-phosphate or oleth-3.

Examples of non-ionic emulsifiers include sorbitan monostearate, glyceryl monostearate, polysorbates, polyethylene derivatives of fatty alcohols, polyoxyethylene ethers of fatty alcohols, such as polyoxyethylene cetyl ether, polyoxyethylene oleyl ether, polyoxyethylene nonylphenyl ether and the like, sorbitan stearate, glyceryl stearate, C₁₂-C₁₈ fatty alcohols, esters and ethers thereof, aliphatic fatty alcohols such as cetyl alcohol or stearyl alcohol or a mixture of the two, fatty alcohols or α-diols oxyethylenated or polyglycerolated such as oleyl alcohol polyoxyethylenated with 10 moles of ethylene oxide, 1,2-octadecanediol polyglycerolated with 2 or 7 moles of glycidol, cyclic fatty alcohols, glycol esters of fatty acids such as ethylene glycol stearate, the monostearates or distearates of glycerol, the polyethylene glycol esters of fatty acids such as polyethylene glycol stearates, the fatty esters of sorbitan oxyethylenated or not and sold under the trade name of Tweens or Spans, the fatty esters of sucrose, the fatty esters of glucose derivatives such as methylglucoside sesquistearate and methylglucoside sesquistearate polyoxyethylenated with 20 moles of ethylene oxide, Arlacel 165 and Myrj 52, fatty alcohols having 10 to 20 carbon atoms, fatty alcohols having 10 to 20 carbon atoms condensed with 2 to 20 moles of ethylene oxide or propylene oxide, alkyl phenols with 6 to 12 carbon atoms in the alkyl chain condensed with 2 to 20 moles of ethylene oxide, mono-fatty acid and di-fatty acid esters of ethylene oxides, mono-fatty acid and di-fatty acid esters of ethylene glycol wherein the fatty acid moiety contains from 10 to 20 carbon atoms, diethylene glycol, polyethylene glycols of molecular weight 200 to 6000, propylene glycols of molecular weight 200 to 3000, glycerol, sorbitol, sorbitan, polyoxyethylene sorbitol, polyoxyethylene sorbitan and hydrophilic wax esters, polyoxyethylene fatty alcohol ethers, polyoxyethylene fatty acid ester, polyoxyethylene sorbitan fatty acid esters, sorbitan fatty acid esters, polyoxyethylene glycol fatty acid esters and polyol fatty acid esters.

Examples of cationic emulsifiers include quaternized ammonium bromide and chloride salts, cetyltrimethylammonium chloride, benzalkonium chloride and cetyl pyridinium chloride, aliphatic amines having fatty chains, e.g., oleylamine and dihydroabietylamine; quaternary ammonium compounds, e.g., lauryl dimethylbenzyl ammonium chloride, amides derived from amino alcohols, e.g., N-aminoethyl oleylamide, n-(stearoyl-colamino-formylmethyl) pyridinium chloride, N-soya-N-ethyl morpholinium ethosulphate, alkyl dimethyl benzyl ammonium chloride, di-isobutylphenoxyethoxyethyl dimethyl benzyl ammonium chloride, cetyl pyridinium chloride, N-(stearoyl-colamino- formylmethyl) pyridinium chloride, N-soya-N-ethyl morpholinium ethosulfate, alkyl dimethyl benzyl ammonium chloride, (diisobutyl-phenoxy-ethoxy) ethyl dimethyl benzyl ammonium chloride, PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, stearamidopropyl dimethyl ammonium lactate, ammonium halides, more especially chlorides and bromides, such as alkyl trimethylammonium chlorides, dialkyl dimethylammonium chlorides and trialkyl methylammonium chlorides, for example, stearyl trimethylammonium chloride, distearyl dimethylammonium chloride, lauryl dimethylammonium chloride, lauryl dimethyl benzylammonium chloride and tricetyl methylammonium chloride, quaternized protein hydrolyzates or protein hydrolyzates derivatized with amino groups which are marketed, for example, under the names Lamequat^{®} and Mackpro^{®}, stearamidopropyl PG-dimonium chloride phosphate, stearamidopropyl ethyldimonium ethosulfate, stearamidopropyl dimethyl (myristyl acetate) ammonium chloride, stearamidopropyl dimethyl cetearyl ammonium tosylate, stearamidopropyl dimethyl ammonium chloride, and stearamidopropyl dimethyl ammonium lactate.

The compositions of the instant invention can comprise a wide range of additional components. The "CTFA Cosmetic Ingredient Handbook", Second edition, 1992, describes a wide variety of cosmetic and pharmaceutical ingredients commonly used in the skin care industry, which are suitable for use in the compositions of the instant invention. Examples of functional classes of ingredients are absorbents, abrasives, anti-acne agents, anticaking agents, antifoaming agents, antimicrobial agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, platicizers, preservatives, propellants, reducing agents, skin bleaching agents, skin conditioning agents (emollients and humectants), skin protectants, solvents, foam boosters, hydrotropes, solubilizing agents, suspending agents (nonsurfactant), sunscreen agents, ultraviolet light absorbers and viscosity increasing agents (aqueous and nonaqueous).

The various starting materials for making a topical preparation are known in the art and reference can be made to known treatises, as well as U.S. Patent Nos. 6,013,271; 6,267,985; 4,992,478; 5,645,854; 5,811,111; and 5,851,543.

The pharmaceutically active agents in the dermatological preparations of interest are fluocinolone acetonide, hydroquinone, and tretinoin.

The amounts of the inert ingredients and active agent(s) in the dermatologic preparation of interest generally are known in the art. It is within the ambit of the artisan to derive particular amounts of the ingredients to obtain a cream of interest.

The particular amount of any one ingredient used is not substantially critical and the amounts used are at the accuracy of the measuring or dispensing means known in the art.

In one embodiment of the invention, approximately 344.8 kg of water, 15.0 kg magnesium aluminum silicate, and 0.2 kg butylated hydroxytoluene are first combined and mixed at 75-80°C to form the aqueous phase. The mixing can be by side scrape agitation at a fixed speed. The resulting aqueous phase is a suspension.

Second, approximately 20.0 kg of cetyl alcohol, 15.0 kg of stearic acid, 20.0 kg of stearyl alcohol, 25.0 kg of methyl gluceth-10, 0.9 kg of methylparaben, 0.1 kg of propylparaben, and 20.0 kg of glycerin are mixed together at medium speed at about 75-80°C to form the non-aqueous phase. The mixing can be at medium speed in a Lightnin^{®} mixer. The resulting non-aqueous phase is a suspension. The second step can be performed before, after or concurrently with the first step.

Then, the non-aqueous phase is added to the aqueous phase and the combined biphasic mixture is cooled to a temperature in the range of 68°C to 72°C, or about 70°C, after which about 17.5 kg of Arlacel^{®} 165, 0.25 kg tretinoin and 0.050 kg fluocinolone acetonide are added and stirred with cooling. When the mixture reaches 60°C, 0.25 kg citric acid is added with mixing and cooling. When the temperature reaches 55°C, 20.0 kg hydroquinone is added with mixing and cooling. When the temperature reaches about 50°C, the mixture is homogenized with a homogenizer, with continued cooling. When the mixture reaches 45°C, 1.0 kg of sodium metabisulfite is added with stirring and cooling. Typically, the sodium metabisulfite is added about 30 minutes after the addition of the hydroquinone. The mixing can be at fixed speed in a side scrape agitator. The resulting composition of matter is an emulsion, i.e., a cream.

The presence of sodium metabisulfite in the cream prevents the oxidation of hydroquinone. The addition of sodium metabisulfite as the cream is cooling advantageously results in a well-mixed composition of matter, with the sodium metabisulfite evenly mixed throughout the cream and preventing the oxidation of the hydroquinone throughout the cream. Another advantage of the process of the invention is that by controlling the temperature at which the components, including hydroquinone, are added, the cream does not turn as brown, resulting in a more pleasing-colored product.

The addition of the emulsifier following the mixing of the non-aqueous and aqueous phases is advantageous for the making of the pharmaceutical composition of the invention. When a standard technique of adding the emulsifier to the non-aqueous phase and then mixing with the aqueous phase was used, no emulsion formed. However, when the emulsifier was added to the mixture of the non-aqueous and aqueous phases with cooling, according to the method of the invention, a useful emulsion did form. This emulsion formed even though the relative proportion of the non-aqueous and aqueous phases according to the successful method of the invention was the same as when an emulsion did not form using the standard technique of adding a non-aqueous phase containing an emulsifier to an aqueous phase.

The resulting TRI-LUMA^{®} Cream contains fluocinolone acetonide, hydroquinone and tretinoin in a hydrophilic cream base for topical application. Each gram of TRI-LUMA^{®} Cream contains as active ingredients, fluocinolone acetonide 0.01% (0.1 mg), hydroquinone 4% (40 mg), and tretinoin 0.05% (0.5 mg), and as inactive ingredients, butylated hydroxytoluene, cetyl alcohol, citric acid, glycerin, glyceryl stearate, magnesium aluminum silicate, methyl gluceth-10, methylparaben, PEG-100 stearate, propylparaben, purified water, sodium metabisulfite, stearic acid, and stearyl alcohol, see TABLE 1.

**TABLE 1**

| **Ingredient** | **500 kg Batch Quantity** | **800 kg Batch Quantity** | **Formula** |
|---|---|---|---|
| magnesium aluminum silicate NF | 15 kg | 24 kg | 3.00% |
| butylated hydroxytoluene NF | 200 g | 320 g | 0.04% |
| cetyl alcohol NF | 20 kg | 32 kg | 4.00% |
| stearic acid NF | 15 kg | 24 kg | 3.00% |
| stearyl alcohol NF | 20 kg | 32 kg | 4.00% |
| methylparaben NF | 900 g | 1,440 g | 0.18% |
| propylparaben NF | 100 g | 160 g | 0.02% |
| Arlacel® 165 [glycerol stearate and PEG-100 stearate glycerol monostearate] | 17.5 kg | 28 kg | 3.50% |
| methyl gluceth-10 | 25 kg | 40 kg | 5.00% |
| glycerin USP | 20 kg | 32 kg | 4.00% |
| tretinoin USP | 250 g | 400 g | 0.05% |
| fluocinolone acetonide USP | 50 g | 80 g | 0.01% |
| citric acid USP | 250 g | 400 g | 0.05% |
| hydroquinone USP | 20 kg | 32 kg | 4.00% |
| sodium metabisulfite NF | 1 kg | 1.6 kg | 0.20% |
| purified water USP | 344.8 kg | 551.6 kg | 68.95% |
| **TOTAL** | | | **100.00%** |

Fluocinolone acetonide is a synthetic fluorinated corticosteroid for topical dermatological use and is classified therapeutically as an anti-inflammatory. It is a white crystalline powder that is odorless and stable in light. The chemical name for fluocinolone acetonide is (6,11,16)-6,9-difluoro-11,21-dihydroxy-16,17-[(1-methylethylidene)bis(oxy)]-pregna-1,-4-diene-3,20-dione. The molecular formula is C₂₄H₃₀F₂O₆ and molecular weight is 452.50.

Hydroquinone is classified therapeutically as a depigmenting agent. It is prepared from the reduction of *p*-benzoquinone with sodium bisulfite. It occurs as fine white needles that darken on exposure to air. The chemical name for hydroquinone is 1,4-benzenediol. The molecular formula is C₆H₆O₂ and molecular weight is 110.11.

Tretinoin is all-*trans*-retinoic acid formed from the oxidation of the aldehyde group of retinene to a carboxyl group. It is highly reactive to light and moisture. Tretinoin is classified therapeutically as a keratolytic. The chemical name for tretinoin is:
(*all*-*E*)-3,7-dimethyl-9-(2,6,6-trimethyl-1-cyclohexen-1-yl)-2,4,6,8-nonatetraenoic acid. The molecular formula is C₂₀H₂₈O₂ and molecular weight is 300.44.

TRI-LUMA™ Cream is typically supplied in 30 g aluminum tubes, NDC 0299-5950-30, and is stored at controlled room temperature 68 to 77°F (20-25 °C).

The details of one or more embodiments of the invention are set forth in the accompanying description above. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this invention belongs.

The following EXAMPLES are presented to more fully illustrate the preferred embodiments of the invention. These EXAMPLES should in no way be construed as limiting the scope of the invention, as defined by the appended claims.

### EXAMPLE I

### HUMAN PHARMACOKINETICS

Percutaneous absorption of unchanged tretinoin, hydroquinone and fluocinolone acetonide into the systemic circulation of two groups of healthy volunteers (Total n=59) was found to be minimal following 8 weeks of daily application of 1 g (Group I, n=45) or 6 g (Group II, n=14) of TRI-LUMA^{®} Cream.

For tretinoin quantifiable plasma concentrations were obtained in 57.78% (26 out of 45) of Group I and 57.14% (8 out of 14) of Group II subjects. The exposure to tretinoin as reflected by the Cₘₐₓ values ranged from 2.01 to 5.34 ng/mL (Group I) and 2.0 to 4.99 ng/mL (Group II). Thus, daily application of TRI-LUMA^{®} Cream resulted in a minimal increase of normal endogenous levels of tretinoin. The circulating tretinoin levels represent only a portion of total tretinoin-associated retinoids, which would include metabolites of tretinoin and that sequestered into peripheral tissues.

For hydroquinone quantifiable plasma concentrations were obtained in 18% (8 out of 44) Group I subjects. The exposure to hydroquinone as reflected by the Cₘₐₓ values ranged from 25.55 to 86.52 ng/mL. All Group II subjects (6g dose) had undetectably low post-dose plasma concentrations.

For fluocinolone acetonide, Groups I and II subjects had undetectably low post-dose plasma concentrations.

The following tests may be helpful in evaluating patients: (a) ACTH or cosyntropin stimulation tests; (b) the A.M. plasma cortisol test; and (c) the urinary free cortisol test.

### EXAMPLE II

### HUMAN CLINICAL STUDIES

Two efficacy and safety studies were conducted in 641 melasma patients between the ages of 21 to 75 years, having skin phototypes I-IV and moderate to severe melasma of the face. TRI-LUMA^{®} Cream was compared with three possible combinations of two of the three active ingredients [(1) hydroquinone 4% (HQ) + tretinoin 0.05% (RA); (2) fluocinolone acetonide 0.01% (FA) + tretinoin 0.05% (RA); (3) fluocinolone acetonide 0.01% (FA) + hydroquinone 4% (HQ)], contained in the same vehicle as TRI-LUMA^{®} Cream.

The patients were instructed to apply their study medication each night, after washing their face with a mild soapless cleanser, for 8 weeks. The patients were also instructed to apply a thin layer of study medication to the hyperpigmented lesion, making sure to cover the entire lesion including the outside borders extending to the normal pigmented skin. The patients were provided a mild moisturizer for use as needed and a sunscreen with SPF 30 for daily use. Moreover, the patients were instructed to avoid sunlight exposure to the face, wear protective clothing Protective clothing and avoidance of sunlight exposure to the face was recommended.

The patients were evaluated for melasma severity at baseline and at weeks 1, 2, 4, and 8 of treatment. Primary efficacy was based on the proportion of patients who had an investigators' assessment of treatment success, defined as the clearing of melasma at the end of the eight-week treatment period. The majority of patients enrolled in the two studies were white (approximately 66%) and female (approximately 98%). TRI-LUMA^{®} Cream was demonstrated to be significantly more effective than any of the other combinations of the active ingredients.

Patients experienced improvement of their melasma with the use of TRI-LUMA^{®} Cream as early as 4 weeks. However, among 7 patients who had clearing at the end of 4 weeks of treatment with TRI-LUMA^{®} Cream, 4 of them did not maintain the remission after an additional 4 weeks of treatment.

After 8 weeks of treatment with the study drug, patients entered into an open-label extension period in which TRI-LUMA^{®} Cream was given on an as-needed basis for the treatment of melasma. In studies, after 8 weeks of treatment with TRI-LUMA^{®} Cream, most patients had at least some improvement. Some had their dark spots clear up completely (38% in one study and 13% in another). In most patients treated with TRI-LUMA^{®} Cream, their melasma came back after treatment. The remission periods appeared to shorten between progressive courses of treatment. Additionally, few patients maintained complete clearing of melasma (approximately 1 to 2%).

**TABLE 2**

| **Investigators' Assessment of Treatment Success* At the End of 8 Weeks of Treatment** | | | | | |
|---|---|---|---|---|---|
| | | **TRI-LUMA^{®} Cream** | **HQ+RA** | **FA+RA** | **FA+HQ** |
| Study No. 1 | Number of Patients | 85 | 83 | 85 | 85 |
| | Number of Successes | 32 | 12 | 0 | 3 |
| | Proportion of Successes | 38% | 15% | 0% | 4% |
| | P-value | | <0.001 | <0.001 | 0.001 |
| Study No. 2 | Number of Patients | 76 | 75 | 76 | 78 |
| | Number of Successes | 10 | 3 | 3 | 1 |
| | Proportion of Successes | 13% | 4% | 4% | 1% |
| | P-value# | | 0.045 | 0.042 | 0.005 |

| | | | | | |
|---|---|---|---|---|---|
| *Treatment success was defined as melasma severity score of zero (melasma lesions cleared of hyperpigmentation). #P-value is from Cochran-Mantel-Haenszel chi-square statistics controlling for pooled investigator and comparing TRI-LUMA^{®} Cream to the other treatment groups. | | | | | |

Based on melasma severity at the beginning of the trial, 161 patients were assessed for improvement at day 56 of treatment. 61% (99 patients) experienced symptom improvement from "moderate" to "mild" or "cleared," and 68% (25) showed improvement from "severe" to "mild" or "cleared" over the 8-week treatment period as shown in TABLE 3.

**TABLE 3**

| **Investigators' Assessment of Change in Melasma Severity from Baseline to Day 56 of Treatment (combined results from studies 1 and 2)** | | | | | | | |
|---|---|---|---|---|---|---|---|
| | | | **Number(%) of Patients at Day 56^{a}** | | | | |
| | **Baseline** | | **Cleared^{b} N(%)** | **Mild^{b} N(%)** | **Moderate^{b} N(%)** | **Severe^{b} N(%)** | **Missin N(%)** |
| | **Severity Rating** | **N** | | | | | |
| Tri-Luma^{®} | Moderate | 124 | 36 (29) | 63 (51) | 18 (16) | 0(0) | 7 (6%) |
| Cream N=161 | Severe | 37 | 6(16) | 19(51) | 9 (24) | 2(5) | 1 (3%) |

| | | | | | | | |
|---|---|---|---|---|---|---|---|
| ^{a} Assessment based on patients with severity scores at day 56. Percentages are based on the total number in the treatment group population. ^{b} Does not include patients who cleared before day 56 or were missing from the day 56 assessment. Assessment scale: Cleared (melasma lesions approximately equivalent to surrounding normal skin or with minimal residual hyperpigmentation); Mild (slightly darker than the surrounding normal skin); Moderate (moderately darker than the surrounding normal skin); Severe (markedly darker than the surrounding normal skin). | | | | | | | |

### EXAMPLE III

### ADVERSE REACTIONS IN HUMANS

In a patch test study to determine sensitization potential in 221 healthy volunteers, three volunteers developed sensitivity reactions to TRI-LUMA^{®} Cream or its components.

In the controlled clinical trials, adverse events were monitored in the 161 patients who used TRI-LUMA^{®} Cream once daily during an 8-week treatment period. There were 102 (63%) patients who experienced at least one treatment-related adverse event during these studies. The most frequently reported events were erythema, desquamation, burning, dryness, and pruritus at the site of application. The majority of these events were mild to moderate in severity. Adverse events reported by at least 1% of patients and judged by the investigators to be reasonably related to treatment with TRI-LUMA^{®} Cream from the controlled clinical studies are summarized (in decreasing order of frequency) as follows:

**TABLE 4**

| **Incidence and Frequency of Treatment-Related Adverse Events with TRI-LUMA**^{®} **Cream In At Least 1% or More of Patients (N=161)** | | |
|---|---|---|
| **Adverse Event** | **Number** | **(%) of Patients** |
| Erythema | 66 | (41%) |
| Desquamation | 61 | (38%) |
| Burning | 29 | (18%) |
| Dryness | 23 | (14%) |
| Pruritus | 18 | (11%) |
| Acne | 8 | (5%) |
| Paresthesia | 5 | (3%) |
| Telangiectasia | 5 | (3%) |
| Hyperesthesia | 3 | (2%) |
| Pigmentary changes | 3 | (2%) |
| Irritation | 3 | (2%) |
| Papules | 2 | (1%) |
| Acne-like rash | 1 | (1%) |
| Rosacea | 1 | (1%) |
| Dry mouth | 1 | (1%) |
| Rash | 1 | (1%) |
| Vesicles | 1 | (1%) |

In an open-label long-term safety study, patients who have had cumulative treatment of melasma with TRI-LUMA^{®} Cream for 6 months showed a similar pattern of adverse events as in the 8-week studies. The following local adverse reactions have been reported infrequently with topical corticosteroids. They may occur more frequently with the use of occlusive dressings, especially with higher potency corticosteroids. These reactions are listed in an approximate decreasing order of occurrence: burning, itching, irritation, dryness, folliculitis, acneiform eruptions, hypopigmentation, perioral dermatitis, allergic contact dermatitis, secondary infection, skin atrophy, striae, and miliaria.

The foregoing description has been presented only for the purposes of illustration and is not intended to limit the invention to the precise form disclosed, but by the claims appended hereto.

## Claims

1. A method of making a topical medicated composition for topical application, the composition being an emulsion comprising water and, as active ingredients, fluocinolone acetonide, hydroquinone, and tretinoin, the method comprising:
(a) mixing water and at least one hydrophilic compound to form an aqueous phase;
(b) mixing at least two hydrophobic compounds to form a non-aqueous phase;
(c) combining the aqueous phase and non-aqueous phase to form a biphasic mixture in the absence of an emulsifier;
(d) mixing fluocinolone acetonide and tretinoin into the mixture of step (c);
(e) mixing at least one emulsifier into the mixture of step (d), or during step (d); and
(f) homogenizing the mixture of step (e) to form the emulsion;
wherein hydroquinone is added in step (d) or after adding the at least one emulsifier.

2. The method of claim 1, wherein said step (a), step (b) or both are conducted at a temperature greater than room temperature to yield a heated phase.

3. The method of claim 2, wherein said heated phase is cooled prior to said combining step (c).

4. The method of claim 2, wherein said heated biphasic mixture is cooled prior to step (d).

5. The method of claim 1, wherein said hydroquinone is added after adding said at least one emulsifier in step (e).

6. The method of claim 1, further comprising adding sodium metabisulfite to said mixture of step (e) after adding said at least one emulsifier.

7. The method of claim 1, wherein said water and said at least one hydrophilic compound are mixed at an elevated temperature.

8. The method of claim 1, wherein said at least two hydrophobic compounds are mixed at an elevated temperature.

9. The method of claim 7 or 8, wherein said elevated temperature is not greater than 80°C.

10. The method of claim 1, wherein said water and said at least one hydrophilic compound are mixed at a temperature of not greater than 80°C, and said at least two hydrophobic compounds are mixed at a temperature of not greater than 80°C.

11. The method of claim 10, wherein said mixture of step (c) is cooled before adding said fluocinolone acetonide and said tretinoin in step (d).

12. The method of claim 10, wherein said mixture of step (d) is cooled while said at least one emulsifier is mixed into said mixture.

13. The method of claim 10, wherein said hydroquinone is added in step (d) following cooling of said mixture.

14. The method of claim 10, wherein said hydroquinone is added in step (e) after said at least one emulsifier is mixed into said mixture.

15. The method of claim 14, wherein said mixture is cooled and sodium metabisulfite is added while said mixture is cooling.

16. The method of claim 1, wherein the aqueous phase comprises water, magnesium aluminum silicate and butylated hydroxytoluene.

17. The method of claim 1, wherein the emulsifier is glyceryl stearate, polyethylene glycol (PEG) stearate or a combination thereof.

18. A topical medicated composition, comprising 0.01 weight % fluocinolone acetonide; 4 weight % hydroquinone; 0.05 weight % tretinoin; 0.04 weight % butylated hydroxy toluene; 4 weight % cetyl alcohol; 0.05 weight % citric acid; 4 weight % glycerin; 3 weight % magnesium aluminum silicate; 5 weight % methyl gluceth; 0.18 weight % methylparaben; 3.5 weight % glyceryl stearate, polyethylene glycol (PEG) stearate or a combination thereof, 0.02 weight % propylparaben, 0.2 weight % sodium metabisulfite, 3 weight % stearic acid and 4 weight % stearyl alcohol.

19. A method according to claim 1 wherein:
(a)said aqueous composition comprising water and at least one hydrophilic compound is prepared while heating at a temperature not greater than 80°C;
(b)said non-aqueous composition comprising at least two hydrophobic compounds is prepared while heating at a temperature not greater than 80°C;
(c) steps (a) and (b) and the mixing of the aqueous and non-aqueous compositions are conducted in the absence of an emulsifier.

20. The method of claim 19, wherein step (c) further comprises cooling the mixture.

21. The method of claim 19, further comprising cooling the mixture after adding at least one emulsifier.

22. The method of claim 19, further comprising cooling the mixture before adding the hydroquinone.

## Patentansprüche

1. Verfahren zum Herstellen einer topischen, mit einem Arzneistoff versetzten Zusammensetzung zur topischen Anwendung, wobei die Zusammensetzung eine Emulsion ist, die Wasser und als Wirkstoffe Fluocinolonacetonid, Hydrochinon und Tretinoin umfasst, wobei das Verfahren Folgendes umfasst:
(a) das Vermischen von Wasser und zumindest einer hydrophilen Verbindung, um eine wässrige Phase zu bilden;
(b) das Vermischen von zumindest zwei hydrophoben Verbindungen, um eine nichtwässrige Phase zu bilden;
(c) das Kombinieren der wässrigen Phase und der nichtwässrigen Phase, um in Abwesenheit eines Emulgators ein biphasisches Gemisch zu bilden;
(d) das Vermischen von Fluocinolonacetonid und Tretinoin mit dem Gemisch aus Schritt (c);
(e) das Vermischen von zumindest einem Emulgator mit dem Gemisch aus Schritt (d) oder während Schritt (d); und
(f) das Homogenisieren des Gemischs aus Schritt (e), um die Emulsion zu bilden;
worin Hydrochinon in Schritt (d) oder nach dem Hinzufügen des zumindest einen Emulgators hinzugefügt wird.

2. Verfahren nach Anspruch 1, worin Schritt (a), Schritt (b) oder beide bei einer Temperatur durchgeführt werden, die höher ist als die Raumtemperatur, um eine erhitzte Phase zu erhalten.

3. Verfahren nach Anspruch 2, worin die erhitzte Phase vor dem Schritt des Kombinierens (c) gekühlt wird.

4. Verfahren nach Anspruch 2, worin das erhitzte biphasische Gemisch vor Schritt (d) gekühlt wird.

5. Verfahren nach Anspruch 1, worin das Hydrochinon nach dem Hinzufügen des zumindest einen Emulgators in Schritt (e) hinzugefügt wird.

6. Verfahren nach Anspruch 1, ferner umfassend das Hinzufügen von Natriummetabisulfit zu dem Gemisch aus Schritt (e) nach dem Hinzufügen des zumindest einen Emulgators.

7. Verfahren nach Anspruch 1, worin das Wasser und die zumindest eine hydrophile Verbindung bei einer erhöhten Temperatur vermischt werden.

8. Verfahren nach Anspruch 1, worin die zumindest zwei hydrophoben Verbindungen bei einer erhöhten Temperatur vermischt werden.

9. Verfahren nach Anspruch 7 oder 8, worin die erhöhte Temperatur nicht höher als 80 °C ist.

10. Verfahren nach Anspruch 1, worin das Wasser und die zumindest eine hydrophile Verbindung bei einer Temperatur von nicht mehr als 80 °C vermischt werden und die zumindest zwei hydrophoben Verbindungen bei einer Temperatur von nicht mehr als 80 °C vermischt werden.

11. Verfahren nach Anspruch 10, worin das Gemisch aus Schritt (c) gekühlt wird, bevor das Fluocinolonacetonid und das Tretinoin in Schritt (d) hinzugefügt werden.

12. Verfahren nach Anspruch 10, worin das Gemisch aus Schritt (d) gekühlt wird, während der zumindest eine Emulgator mit dem Gemisch vermischt wird.

13. Verfahren nach Anspruch 10, worin das Hydrochinon in Schritt (d) nach dem Kühlen des Gemischs hinzugefügt wird.

14. Verfahren nach Anspruch 10, worin das Hydrochinon in Schritt (e) hinzugefügt wird, nachdem der zumindest eine Emulgator mit dem Gemisch vermischt wurde.

15. Verfahren nach Anspruch 14, worin das Gemisch gekühlt wird und Natriummetabisulfit hinzugefügt wird, während das Gemisch abkühlt.

16. Verfahren nach Anspruch 1, worin die wässrige Phase Wasser, Magnesiumaluminiumsilikat und butyliertes Hydroxytoluol umfasst.

17. Verfahren nach Anspruch 1, worin der Emulgator Gylcerylstearat, Polyethylenglykol- (PEG-) Stearat oder eine Kombination davon ist.

18. Topische, mit einem Arzneistoff versetzte Zusammensetzung, umfassend 0,01 Gew.-% Fluocinolonacetonid; 4 Gew.-% Hydrochinon, 0,05 Gew.-% Tretinoin; 0,04 Gew.-% butyliertes Hydroxytoluol; 4 Gew.-% Cetylalkohol; 0,05 Gew.-% Citronensäure; 4 Gew.-% Glycerin; 3 Gew.-% Magnesiumaluminiumsilikat; 5 Gew.-% Methylgluceth; 0,18 Gew.-% Methylparaben; 3,5 Gew.-% Glycerylstearat, Polyethylenglykol-(PEG-) Stearat oder eine Kombination davon, 0,02 Gew.-% Propylparaben, 0,2 Gew.-% Natriummetabisulfit, 3 Gew.-% Stearinsäure und 4 Gew.-% Stearylalkohol.

19. Verfahren nach Anspruch 1, worin:
(a) die wässrige Zusammensetzung, die Wasser und zumindest eine hydrophile Verbindung umfasst, hergestellt wird, während sie bei einer Temperatur von nicht mehr als 80 °C erhitzt wird;
(b) die nichtwässrige Zusammensetzung, die zumindest zwei hydrophobe Verbindungen umfasst, hergestellt wird, während sie bei einer Temperatur von nicht mehr als 80 °C erhitzt wird;
(c) die Schritte (a) und (b) und das Vermischen der wässrigen und der nichtwässrigen Zusammensetzung in Abwesenheit eines Emulgators durchgeführt werden.

20. Verfahren nach Anspruch 19, worin Schritt (c) ferner das Kühlen des Gemischs umfasst.

21. Verfahren nach Anspruch 19, ferner umfassend das Kühlen des Gemischs nachdem zumindest ein Emulgator hinzugefügt wurde.

22. Verfahren nach Anspruch 19, ferner umfassend das Kühlen des Gemischs bevor das Hydrochinon hinzugefügt wird.

## Revendications

1. Procédé de préparation d'une composition médicamenteuse topique pour application topique, la composition étant une émulsion comprenant de l'eau et, en tant que principes actifs, de l'acétonide de fluocinolone, de l'hydroquinone, et de la trétinoïne, le procédé comprenant les étapes consistant à :
(a) mélanger de l'eau et au moins un composé hydrophile pour former une phase aqueuse ;
(b) mélanger au moins deux composés hydrophobes pour former une phase non aqueuse ;
(c) combiner la phase aqueuse et la phase non aqueuse pour former un mélange biphasique en l'absence d'émulsifiant ;
(d) mélanger de l'acétonide de fluocinolone et de la trétinoïne dans le mélange de l'étape (c) ;
(e) mélanger au moins un émulsifiant dans le mélange de l'étape (d), ou pendant l'étape (d) ; et
(f) homogénéiser le mélange de l'étape (e) pour former l'émulsion ;
dans lequel l'hydroquinone est ajoutée dans l'étape (d) ou après l'ajout dudit au moins un émulsifiant.

2. Procédé selon la revendication 1, dans lequel ladite étape (a), étape (b) ou les deux sont conduites à une température supérieure à la température ambiante pour donner une phase chauffée.

3. Procédé selon la revendication 2, dans lequel ladite phase chauffée est refroidie avant ladite étape de combinaison (c).

4. Procédé selon la revendication 2, dans lequel ledit mélange biphasique chauffé est refroidi avant l'étape (d).

5. Procédé selon la revendication 1, dans lequel ladite hydroquinone est ajoutée après l'ajout dudit au moins un émulsifiant dans l'étape (e).

6. Procédé selon la revendication 1, comprenant en outre l'ajout de métabisulfite de sodium audit mélange de l'étape (e) après l'ajout dudit au moins un émulsifiant.

7. Procédé selon la revendication 1, dans lequel ladite eau et ledit au moins un composé hydrophile sont mélangés à une température élevée.

8. Procédé selon la revendication 1, dans lequel lesdits au moins deux composés hydrophobes sont mélangés à une température élevée.

9. Procédé selon la revendication 7 ou 8, dans lequel ladite température élevée n'est pas supérieure à 80 °C.

10. Procédé selon la revendication 1, dans laquelle ladite eau et ledit au moins un composé hydrophile sont mélangés à une température non supérieure à 80 °C, et lesdits au moins deux composés hydrophobes sont mélangés à une température non supérieure à 80 °C.

11. Procédé selon la revendication 10, dans lequel ledit mélange de l'étape (c) est refroidi avant l'ajout dudit acétonide de fluocinolone et de ladite trétinoïne dans l'étape (d).

12. Procédé selon la revendication 10, dans lequel ledit mélange de l'étape (d) est refroidi pendant que ledit au moins un émulsifiant est mélangé dans ledit mélange.

13. Procédé selon la revendication 10, dans lequel ladite hydroquinone est ajoutée dans l'étape (d) à la suite du refroidissement dudit mélange.

14. Procédé selon la revendication 10, dans lequel ladite hydroquinone est ajoutée dans l'étape (e) après que ledit au moins un émulsifiant est mélangé dans ledit mélange.

15. Procédé selon la revendication 14, dans lequel ledit mélange est refroidi et du métabisulfite de sodium est ajouté pendant que ledit mélange refroidit.

16. Procédé selon la revendication 1, dans lequel la phase aqueuse comprend de l'eau, de l'aluminosilicate de magnésium et de l'hydroxytoluène butylé.

17. Procédé selon la revendication 1, dans lequel l'émulsifiant est le stéarate de glycéryle, le stéarate de polyéthylèneglycol (PEG) ou une combinaison de ceux-ci.

18. Composition médicamenteuse topique, comprenant 0,01 % en poids d'acétonide de fluocinolone ; 4 % en poids d'hydroquinone ; 0,05 % en poids de trétinoïne ; 0,04 % en poids d'hydroxytoluène butylé ; 4 % en poids d'alcool cétylique ; 0,05 % en poids d'acide citrique ; 4 % en poids de glycérine ; 3 % en poids d'aluminosilicate de magnésium ; 5 % en poids de méthyl gluceth ; 0,18 % en poids de méthylparabène ; 3,5 % en poids de stéarate de glycéryle, de stéarate de polyéthylèneglycol (PEG) ou d'une combinaison de ceux-ci, 0,02 % en poids de propylparabène, 0,2 % en poids de métabisulfite de sodium, 3 % en poids d'acide stéarique et 4 % en poids d'alcool stéarylique.

19. Procédé selon la revendication 1, dans lequel :
(a) on prépare ladite composition aqueuse comprenant de l'eau et au moins un composé hydrophile tout en chauffant à une température non supérieure à 80 °C ;
(b) on prépare ladite composition non aqueuse comprenant au moins deux composés hydrophobes tout en chauffant à une température non supérieure à 80 °C ;
(c) les étapes (a) et (b) et le mélange des compositions aqueuse et non aqueuse sont conduits en l'absence d'émulsifiant.

20. Procédé selon la revendication 19, dans lequel l'étape (c) comprend en outre le refroidissement du mélange.

21. Procédé selon la revendication 19, comprenant en outre le refroidissement du mélange après l'ajout d'au moins un émulsifiant.

22. Procédé selon la revendication 19, comprenant en outre le refroidissement du mélange avant l'ajout de l'hydroquinone.
